# EUROPEAN PATENT APPLICATION

(11) **EP 2 659 883 A1**
(43) Date of publication of application: **06.11.2013**
(21) Application number: 11844778.8
(22) Date of filing: 28.11.2011
(51) Int. Cl.: A61K 9/50, A61K 35/12, C12N 11/10

(54) **MICROCAPSULE PREPARATION OF ALGINATE-CHITOSAN ACYL DERIVATIVES, PREPARATION AND APPLICATION THEREOF**

(30) Priority: 30.11.2010 CN 201010566996
(71) Applicant: Dalian Institute Of Chemical Physics, Chinese Academy Of Sciences, Liaoning 116023 (CN)
(72) Inventor: MA, Xiaojun, Dalian Liaoning 116023 (CN); YU, Weiting, Dalian Liaoning 116023 (CN); XIE, Hongguo, Dalian Liaoning 116023 (CN); LIU, Xiudong, Dalian Liaoning 116023 (CN); XIE, Weiyang, Dalian Liaoning 116023 (CN); ZHENG, Guoshuang, Dalian Liaoning 116023 (CN)
(74) Representative: Mattsson, Niklas
(86) International application number: PCT/CN2011/083023
(87) International publication number: WO 2012/072012

(57) **Abstract**

The present invention relates to a microcapsule preparation product of alginate-chitosan acyl derivatives, which is produced by mixing microcapsules of alginate-chitosan acyl derivatives with an aqueous solution, wherein the biomicrocapsule structureconsists of two parts, a microcapsule membrane and an inner core; the microcapsule membrane is a polyelectrolyte composite hydrogel membrane formed by chitosan, alginates and chitosan acyl derivatives, and the inner core is an alginate liquid or a hydrogel environment containing cells.

## Description

### BACKGROUND OF THE INVENTION

### 1. Technical Field

The present invention relates to a microcapsule product, in particular to a microcapsule product of alginate-chitosan acyl derivatives for living cell encapsulation.

### 2. Description of Related Art

In 1960s, Chang reported a semipermeable microcapsule capable of encapsulating bioactive substances (such as proteins and enzymes) and cells to maintain the activity of the biological substances *[see* Chang TMS. Semipermeable microcapsules, Science, 1964, 146:524-525]. In early of 1980s, for tissue/cell function defect diseases (such as the diabetes), Lim and Sun successfully prepared sodium alginate/α-polylysine semipermeable microcapsules (α-APA microcapsule for short). With Wistar rat islet cells encapsulated, the α-APA microcapsule was transplanted in the Wistar Lewis rats with diabetes, and then insulin was secreted and released to regulate the blood sugar *[see* Lim F, Sun A M. Microencapsulated islets bioartificial endocrine pancreas, Science, 1980, 210:908-910]. This drove the rapid development of the study on the materials and preparation methods related to microcapsule technology, which has been widely applied in the preclinical research of the biomedical fields such as the cell transplantation, drug delivery, and gene therapy *[see* Wang W, Liu XD, Ma XJ, et al. Microencapsulation using natural polysaccharides for drug delivery and cell implantation, J. Mater. Chem., 2006, 16:3252-3267]. Among the numerical biomicrocapsules, sodium alginate-polylysine (α-APA) microcapsules are widely applied. However, the high price (300-400 US$/g), poor inherent biocompatibility and toxicity of polylysine greatly limit the clinical application of α-APA microcapsules [see Strand B L, Ryan TL, Veld P I, et al. Cell Transplant., 2001, 10:263-275]. Chitosan, a natural polysaccharide, has been used as a substitute for polylysine to prepare microcapsules for cell encapsulation due to its low cost, good membrane-forming property, and high membrane mechanical strength [*see* L. Baruch, M. Machluf, Alginate-chitosan complex coacervation for cell encapsulation: Effect on mechanical properties and on long-term viability, Biopolymers 82 (2006):570-579]. However, the existing chitosan microcapsule for cell encapsulation has high surface roughness and surface charges, which easily *cause in* vivo protein absorption after transplanted in a body and further arouse fibrosis of the body.

### BRIEF SUMMARY OF THE INVENTION

To solve the above problem, the present invention provides a microcapsule of alginate-chitosan acyl derivatives, preparation and application thereof.

### Technical solution:

By using the acylated chitosan for preparing the biomicrocapsule, the present invention develops a novel polyelectrolyte complex microcapsule product of alginate-chitosan acyl derivatives for encapsulation of bioactive substances. The novel microcapsule product can not only solve the problems of surface roughness and surface charge, but also maintain the strength and immunoisolation performance of the microcapsule. The microcapsule preparation of alginate-chitosan acyl derivatives of the present invention is produced by mixing microcapsules of alginate-chitosan acyl derivatives with aqueous solution, wherein:
the biomicrocapsule structure consists of two parts, a microcapsule membrane and an inner core; the microcapsule membrane is a polyelectrolyte composite hydrogel membrane formed by chitosan, alginate, and chitosan acyl derivatives, and the inner core is an alginate liquid or a hydrogel environment containing cells.

In the biomicrocapsule preparation product of the present invention, the microcapsules are spherical microcapsules with a particle size of 10 to 2,000µm; the membrane has a thickness of 0.1 to 100 µm, and the molecular weight of the alginate forming the membrane is 10 kDa to 2,000 kDa (e.g., 50 kDa to 200 kDa; 200 kDa to 500 kDa; 600 kDa to 1,000 kDa; 1,000 kDa to 2,000 kDa); the chitosan material has a degree of deacetylation of 70-98%, and molecular weight of 1 kDa to 500 kDa (e.g., 1 kDa to 50 kDa;10 kDa to 100 kDa; 120 kDa to 300 kDa; 350kDa to 500kDa); the molecular weight of the chitosan acyl derivatives is 1kDa∼800kDa (e.g., 1 kDa to 50 kDa; 10 kDa to 100 kDa; 120 kDa to 300 kDa; 350kDa to 500kDa); the mass ratio of chitosan, alginate and chitosan acyl derivatives is 0:1:0.1 to 10:1:10; the alginate concentration in the core is 0.1 to 50g/L, and the cells in the core accounts for 10 to 98 v/v %.

In the biomicrocapsule preparation product, the chitosan acyl derivatives in the microcapsule are N-acyl chitosan, with a monomer structure as below: 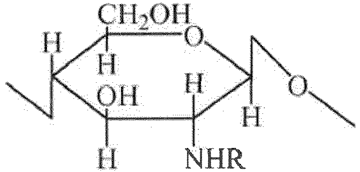 wherein, -R represents formyl, acetyl, propionyl, butyryl, valeryl or caproyl; the substitution value of the acyl derivatives is 10 to 60%; the molecular weight of the chitosan framework material is 1 to 400 kDa; and the degree of deacetylation is 90 to 98%.

In the biomicrocapsule preparation product, the alginate as a component of the microcapsule membrane is potassium or sodium alginate.

In the biomicrocapsule preparation product, the alginate gel in the core of the microcapsule is an alginate hydrogel of one or two or more of divalent calcium, barium or zinc, and the alginate liquid is the solution of potassium or sodium alginate.

In the biomicrocapsule preparation product, the volume ratio of the biomicrocapsule to the aqueous solution is 10:1 to 1:100, wherein the aqueous solution is one or a mixture of two or more of normal saline, HEPES solution, hyaluronic acid solution with an apparent viscosity of 5 to 2,000cp (25°C, refer to the apparent viscosity measured at a temperature of 25°C), the sodium alginate with an apparent viscosity of 5 to 2,000cp (25°C), the glucosan solution with an apparent viscosity of 5 to 2,000cp (25°C), glycerol solution with an apparent viscosity of 5 to 2,000cp (25°C), polyethylene glycol solution with an apparent viscosity of 5 to 2,000cp (25°C), polyvinylpyrrolidone solution with an apparent viscosity of 5 to 2,000cp (25°C), cellulose derivative solution with an apparent viscosity of 5 to 2,000cp (25°C), cyclodextrin solution with an apparent viscosity of 5 to 2,000cp (25°C), starch solution with an apparent viscosity of 5 to 2,000cp (25°C), and starch derivative solution with an apparent viscosity of 5 to 2,000cp (25°C).

In the biomicrocapsule preparation product, the microcapsule membrane is a hydrogel membrane formed by chitosan, alginate, and chitosan acyl derivatives through polyelectrolyte complexation reaction. The preparation steps of the product are as follows: under the sterilized conditions:
1) preparing alginate gel microspheres encapsulating living cells, called microspheres A;
2) soaking the microspheres A obtained in step 1) into the chitosan solution in a volume ratio of 1:1 to 1:40 (i.e., microspheres A : chitosan solution (v/v)), allowing them to react for 1 to 60 min to obtain sodium alginate-chitosan microcapsules called microspheres B, and separating and washing the microspheres B with normal saline; wherein
   the chitosan solution is prepared by dissolving the chitosan in the acetic acid-sodium acetate buffer solution with a pH of 5.5 to 7.0, and the chitosan concentration is 0.1 to 15 g/L;
3) soaking the microspheres B obtained in step 2) into the alkaline metal alginate solution (the alginate concentration is 0.1 to 5 g/L) in a volume ratio of 1:1 to 1:40 (i.e., microspheres B : alkaline metal alginate solution (v/v)), allowing them to react for 1 to 60 min to obtain microcapsules called microspheres C, and separating and washing the microspheres C with normal saline;
4) repeating step 2) and step 3) for 1-5 cycles to obtain microcapsules called microspheres D, and separating and washing the microspheres D with normal saline;
5) soaking the microspheres A, B, C or D respectively obtained in step 1), 2), 3) or 4) into the chitosan acyl derivative solution in a volume ratio of 1:1 to 1:40 (i.e., microspheres : chitosan acyl derivative solution (v/v)), allowing them to react for 1 to 60 min to obtain microcapsules having an inner gel core, called microspheres E, and separating and washing the microspheres E with the saline, wherein
   the chitosan acyl derivative solution is prepared by dissolving the chitosan acyl derivatives in the normal saline, HEPES buffer solution, PBS buffer solution or acetic acid-sodium acetate buffer solution with a pH of 5.5 to 7.0, and the chitosan acyl derivative concentration is 0.1 to 20 g/L;
6) soaking the microspheres E obtained in step 5) into the alkaline metal alginate solution, and repeat step 3) to obtain microcapsules with neutral surface and the inner gel core, called microspheres F;
7) soaking the microspheres F obtained in step 6) into the organic metal chelating agent solution in a volume ratio of 1:1 to 1:40 (i.e., microspheres F : the organic metal chelating agent solution (v/v)) to liquefy the alginate gel in the microcapsules, allowing them to react for 1 to 60 min, seperating the product, washing it with normal saline to obtain the microcapsules having an inner liquid core, called microspheres G;
8) mixing the microspheres E, F or G respectively obtained in step 5), 6) or 7) with above aqueous solution to obtain the microcapsule preparation of alginate-chitosan acyl derivatives.

The alginate gel microspheres are alginate hydrogel of one or two or more of divalent calcium, barium or zinc.

The alkaline metal alginate for neutralizing the surface charges is potassium or sodium alginate with a molecular weight of 10kDa to 2,000kDa and a concentration of 0.1 to 5g/L.

The organic metal-chelating agent solution involved in the liquefying reaction is 40 to 70mmol/L of sodium citrate or 50 to 200mmol/L of EDTA.

In the biomicrocapsule preparation of the present invention, the microcapsules are used for cell encapsulation.

Wherein, the cells may be *ex vivo* or *in vitro* cells from human or mammals, such as islet cells, liver cells, thyroid cells, parathyroid cells, adrenal chromaffin cells, cells capable of secreting bioactive substances, cell lines cells, genetically engineered cells, stem cells or various differentiated cells from stem cells.

The invention has the following advantages:
1. Compared with the traditional sodium alginate-polylysine (APA) microcapsules and sodium alginate-chitosan (ACA) microcapsules, the novel microcapsule product of alginate-chitosan acyl derivatives provided by the present invention shows higher biocompatibility because the surface roughness of the microcapsule membrane is significantly lower than that of APA microcapsules and that of the ACA microcapsules.
2. While maintaining excellent biocompatibility, the microcapsule membrane of the product of the present invention has outstanding membrane strength, capable of maintaining the intactness when applied in the tissue/cell transplantation and cell culture.
3. The microcapsules of the present invention have excellent immunoisolation performance, capable of maintaining immunoisolation when applied in heterotransplantation of tissue/cell. The cells encapsulated in the microcapsule cannot exit from the microcapsules, the antibody molecules, complement molecules, and immune cells outside the microcapsules cannot enter the microcapsules to kill the cells, meanwhile the active substances secreted during the cellular metabolism can freely enter and leave the microcapsules.
4. The process and conditions for preparing the product of the present invention are mild; and the chitosan acyl derivatives are dissolvable in normal saline, which is good for maintaining the activity of the cells.

### BRIEF DESCRIPTION OF THE DRAWINGS

Figure 1 shows the comparison results of the surface roughness of the alginate-chitosan acyl derivatives (AC_{acety}l) membrane, AC membrane and AP membrane in Example 1, Comparative Example 1 and Comparative Example 2.
Figure 2 is an optical photo of microcapsules recovered from the abdominal cavity of the mouse after the transplantation of the novel microcapsule preparation product of alginate-chitosan-chitosan acyl derivatives for one month in Example 1 (the scale in the figure is 100 µm).
Figure 3 is an optical photo of microcapsules recovered from the abdominal cavity of the mouse one month after the transplantation of the traditional ACA microcapsules in the Comparative Example 1 (the scale in the figure is 100 µm).

### SPECIFIC MODE FOR CARRYING OUT THE INVENTION

Means for forming the alginate gel microspheres include the electrostatic droplet method (*see* In Vivo Culture of Encapsulated Endostatin-Secreting Chinese Hamster Ovary Cells for Systemic Tumor Inhibition. Human Gene Therapy. 2007, 18:474-481), orifice extrusion method (*see* Chinese Application No. 200510136769.7 filed by the same applicant, titled as "A Method for Preparing Micro-spherical Initial Fish Baits for Highly Economic Fishes"), emulsification-external gelation method (see Preparation of lactic acid bacteria-enclosing alginate beads in emulsion system: effect of preparation parameters on bead characteristics, Polym. Bull., 2009, 63:599-607), emulsification-internal gelation method (*see* Emulsification-internal Gelation Process for Preparation of Immobilized Yeast Microcapsules, CIESC Journal, 2009, 60(3):710-717) or membrane emulsification method (*see* Preparation of uniform calcium alginate gel beads by membrane emulsification coupled with internal gelation. Journal of Applied Polymer Science, 2003, 87(5):848-852).

### Example 1

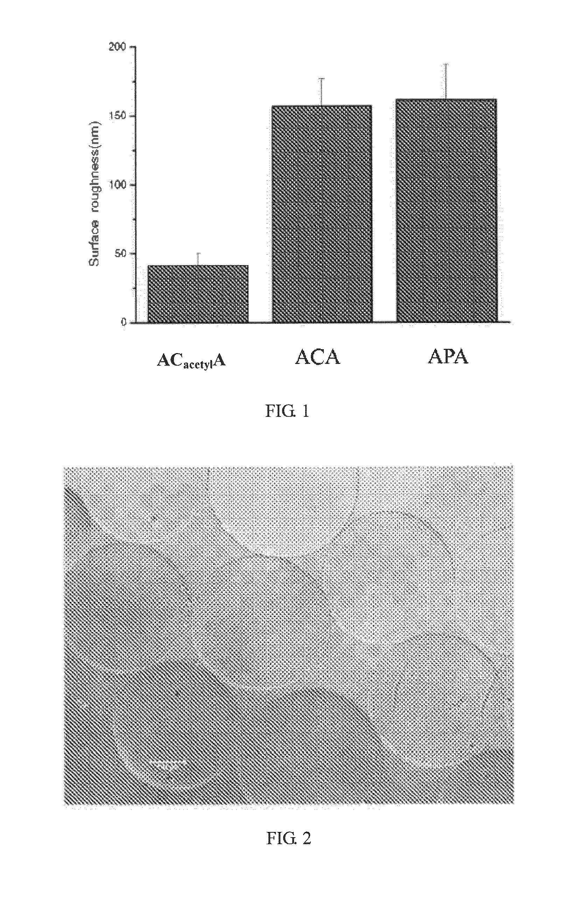

1) Prepare the calcium alginate gel microspheres by a high-voltage electrostatic method under sterilized conditions.
2) Soak the microspheres into acetyl modified chitosan solution (the molecular weight of the chitosan framework is 50kDa; the substitution value of acetyl is 40%; and the solution is prepared by normal saline, with a concentration of 5g/L) in a volume ratio of 1:10 (i.e., the microspheres : the chitosan solution (v/v)), allow them to react for 20 min, wash with normal saline, and then allow the product to react with 2g/L of sodium alginate solution for 10 min, wash with normal saline to obtain AC_{acetyl}A microcapsules.
3) Measure the surface roughness of the resultant AC_{acetyl}A polyelectrolyte complex membrane with a surface profiler, and the result shows that the minimum surface roughness of the membrane is 42±9nm, which is obviously lower than that of the APA membrane and that of the ACA membrane (see Figure 1) prepared by the same method in the Comparative Examples.
4) Mix the AC_{acetyl}A microcapsules with normal saline in a volume ratio of 1:2, transplant the mixture into the abdominal cavity of a mouse with a syringe, and recover them after one month. It is found that the microcapsules can be removed by just washing the abdominal cavity of the mouse with normal saline; they still have high strength and are intact; and the surface of the microcapsule is smooth and free from fibrous encapulation (see Figure 2).

### Comparative Example 1

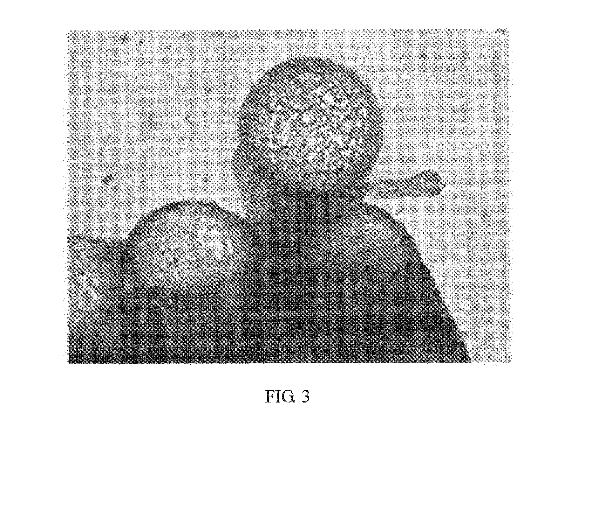

1) Soak the calcium alginate gel microspheres prepared in Example 1 into a chitosan solution (the molecular weight of the chitosan is 50kDa; the degree of deacetylation is 95%; the chitosan is dissolved in the acetic acid-sodium acetate buffer solution with a pH of 6.5; and the concentration of chitosan is 5g/L) in a volume ratio of 1:10 (i.e., the microspheres : the chitosan solution (v/v)), allow them to react for 20 min, wash with normal saline, then allow the washed product to react with 0.2% of sodium alginate solution for 10 min, and wash with normal saline to obtain the ACA microcapsules.
2) Measure the surface roughness of the ACA polyelectrolyte complex membrane with a surface profiler, and the result shows that the surface roughness of the membrane is 157±20, which is obviously higher than that of the AC_{acetyl}A polyelectrolyte complex membrane (see Figure 1) produced in Example 1.
3) Mix the ACA microcapsules with normal saline in a volume ratio of 1: 2, transplant the mixture into the abdominal cavity of a mouse with a syringe, and recover them after one month. The results show that, it is difficult to remove the ACA microcapsules by washing the abdominal cavity of the mouse with normal saline; and the microcapsule surface shows obvious fibrous encapulation (see Figure 3).

### Comparative Example 2

1) Soak the calcium alginate gel microspheres prepared in Example 1 into the polylysine solution (the molecular weight of the polylysine is 20kDa; and the concentration is 5g/L) in a volume ratio of 1:10 (i.e., the microspheres : the polylysine solution (v/v)), allow them to react for 20 min, wash with normal saline, then allow the washed product to react with 2g/L of sodium alginate solution for 10 min, and wash with normal saline to obtain the APA microcapsules.
2) Measure the surface roughness of the APA polyelectrolyte complex membrane with a surface profiler, and the result shows that the surface roughness of the APA membrane is 161±26, which is obviously higher than that of the AC_{acetyl}A polyelectrolyte complex membrane (see Figure 1) produced in Example 1.

### Example 2

1) Prepare the calcium alginate gel microspheres encapsulating the porcine liver cells by an orifice extrusion method, wherein the cell content in the microspheres is 5×10⁷ cells/mL microspheres.
2) Soak the microspheres into the chitosan solution (the molecular weight of the chitosan is 20kDa; the degree of deacetylation is 90%; the chitosan is dissolved in the acetic acid-sodium acetate buffer solution with a pH of 6.8; and the concentration of chitosan is 4g/L) and the acyl modified chitosan solution (the molecular weight of the chitosan framework is 60kDa; the degree of acyl substitution is 30%; and the chitosan is dissolved in the acetic acid-sodium acetate solution with a pH of 6.8; and the concentration is 4g/L) in a volume ratio of 1:10 (i.e., the microspheres : the chitosan solution or the acyl modified chitosan solution (v/v)) in sequence, allow them to react for 20 min, wash with normal saline, then allow the washed product to react with the 0.2% of sodium alginate solution for 10 min, and wash with normal saline to obtain the ACC_{formyl}A microcapsules.
3) Prepare an extracorporeal artificial liver system with the obtained ACC_{formyl}A microcapsules encapsulating porcine liver cells and apply the system to a model dog with liver failure. The results show that, the glutamic-pyruvic transaminase and glutamic-oxalacetic transaminease levels of the mouse with the liver failure recover to the normal ones; the blood ammonia indicators return to normal; the liver failure symptom of the dog is eliminated; the ACC_{formyl}A microcapsules keep intact in the artificial liver system; and the protein absorption phenomenon is not found after blood perfusion.

### Example 3

1) Prepare the calcium alginate gel microspheres encapsulating the porcine islet cells by a high-voltage electrostatic method, wherein each microsphere contains 1-2 islet cells.
2) Soak the microspheres into the chitosan solution (the molecular weight of the chitosan is 40kDa; the degree of deacetylation is 98%; the chitosan is dissolved in the acetic acid-sodium acetate buffer solution with a pH of 6.5; and the concentration is 5g/L), 0.2% of sodium alginate solution and the acetyl modified chitosan solution (the molecular weight of the chitosan framework is 60kDa; the degree of acetyl substitution is 50%; the acetyl modified chitosan is dissolved in normal saline; and the concentration is 5g/L) in a volume ratio of 1:10 (i.e., the microspheres : each solution (v/v)) in sequence, allow them to react for 20 min, wash with normal saline, then allow the washed product to react with 2g/L of sodium alginate solution for 10 min, wash with normal saline, liquefy with 55mM of sodium citrate solution, and wash with normal saline to obtain the ACC_{acetyl}A microcapsules.
3) After mixing the resultant ACC_{acetyl}A microcapsules with the HEPES solution in a volume ratio of 1:5, apply the mixture for cell therapy of the diabetes model rat. By transplantation in the abdominal cavity, the blood sugar level of the rat returns to normal one day after the transplantation, and the diabetes symptom is obviously improved; the microcapsules are recovered six months after *in vivo* transplantation, it is found that, the microcapsules are intact; the microcapsule surface is smooth and free from fibrous encapsulation; and the islet cells in the microcapsules keep alive with positive dithizone staining.

### Example 4

1) Prepare the calcium alginate gel microspheres encapsulating the rat thyroid cells by a high-voltage electrostatic method, wherein the cell content in the microspheres is 3 × 10⁷ cells/mL microspheres.
2) Soak the microspheres into the chitosan solution (the molecular weight of the chitosan is 100kDa; the degree of deacetylation is 95%; the chitosan is dissolved in the acetic acid-sodium acetate buffer solution with a pH of 6.0; and the concentration is 5g/L) and the propionyl modified chitosan solution (the molecular weight of the chitosan framework is 20kDa; the degree of propionyl substitution is 40%; and the propionyl modified chitosan is dissolved in the PBS buffer solution; and the concentration is 5g/L) in a volume ratio of 1:10 (i.e., the microspheres : each solution (v/v)) in sequence, allow them to react for 20 min, wash with normal saline, then allow the washed product to react with 2g/L of sodium alginate solution for 10 min, wash with normal saline, liquefy with the 55mM of sodium citrate solution, and wash with normal saline to obtain the ACC_{propionyl}A microcapsules.
3) After mixing the resultant ACC_{propionyl}A microcapsules encapsulating the rat thyroid cells with a hyaluronic acid solution (20g/L) with an apparent viscosity of 500cp (25°C) in a volume ratio of 1:1, transplant the mixture at the deltoid of the heterogeneous model rat with hypothyroidism. The results show that, the hypothyroidism symptom of the rat is eliminated; T3 and T4 levels return to normal; the ACC_{propionyl}A microcapsules keep intact and the surface is free from fibrosis, when they are recovered three months after transplantation.

### Example 5

1) Prepare the calcium alginate gel microspheres encapsulating the bovine adrenal chromaffin cells by a high-voltage electrostatic method, wherein the cell content in the microspheres is 2×10⁷ cells/mL microspheres.
2) Soak the microspheres into the chitosan solution (the molecular weight of the chitosan is 10kDa; the degree of deacetylation is 90%; the chitosan is dissolved in the acetic acid-sodium acetate buffer solution with a pH of 6.8; and the concentration is 5g/L) and the butyryl modified chitosan solution (the molecular weight of the chitosan framework is 10kDa; the degree of butyryl substitution is 30%; and the butyryl modified chitosan is dissolved in the acetic acid-sodium acetate buffer solution with a pH of 6.8; and the concentration is 5g/L) in a volume ratio of 1:10 (i.e., the microspheres : each solution (v/v)) in sequence, allow them to react for 20 min, wash with normal saline, then allow the washed product to react with 2g/L of sodium alginate solution for 10 min, wash with normal saline, liquefy with 55mM of sodium citrate solution, and wash with normal saline to obtain the ACC_{butyryl}A microcapsules.
3) After mixing the resultant ACC_{butyryl}A microcapsules encapsulating the bovine adrenal chromaffin cells with a sodium alginate solution (20g/L) with an apparent viscosity of 1000cp (25°C) in a volume ratio of 1:1, transplant the mixture at a fixed point of the skull of a model monkey with the Parkinson's disease. The results show that, the Parkinson symptoms such as hemiplegia of the monkey suffering from said disease areeliminated; the ACC_{butyryl}A microcapsules keep intact, and the surface is free from fibrous encapsulation, when they are recovered six months after transplantation,.

### Example 6

1) Prepare the calcium alginate gel microspheres encapsulating the bovine adrenal chromaffin cells by a high-voltage electrostatic method, wherein the cell content in the microspheres is 1×10⁷ cells/mL microspheres.
2) Soak the microspheres into the chitosan solution (the molecular weight of the chitosan is 70kDa; the degree of deacetylation is 98%; the chitosan is dissolved in the acetic acid-sodium acetate buffer solution with a pH of 6.3; and the concentration is 5g/L), 0.2% of sodium alginate solution and valeryl modified chitosan solution (the molecular weight of the chitosan framework is 20kDa; the degree of valeryl substitution is 40%; and the valeryl modified chitosan is dissolved in HEPES buffer solution; and the concentration is 5g/L) in a volume ratio of 1:10 (the microspheres : each solution (v/v)) in sequence, allow them to react for 20 min, wash with normal saline, then allow the washed product to react with 2g/L of sodium alginate solution for 10 min, and wash with normal saline to obtain the ACC_{valeryl}A microcapsules.
3) After mixing the resultant ACC_{valeryl}A microcapsules encapsulating the bovine adrenal chromaffin cells with sodium alginate solution (30g/L) with an apparent viscosity of 800 cp (25°C) in a volume ratio of 1:1, transplant the mixture to the subarachnoid space of spinal cord of the model rat with the intractable pain. The results show that, the intractable pain symptom of the rat is relieved; the number of limb movement is significantly reduced; the ACC_{valeryl}A microcapsules keep intact, and the surface is free from fibrous encapsulation, when they are recovered six months after transplantation.

### Example 7

1) Prepare the calcium alginate gel microspheres encapsulating CHO cells containing recombinant vascular endothelial endostatin by a high-voltage electrostatic method, wherein the cell content in the microspheres is 5×10⁷ cells /mL microspheres
2) Soak the microspheres into the chitosan solution (the molecular weight of the chitosan is 20kDa; the degree of deacetylation is 92%; the chitosan is dissolved in the acetic acid-sodium acetate buffer solution with a pH of 6.5; and the chitosan concentration is 5g/L) and caproyl modified chitosan solution (the molecular weight of the chitosan framework is 20kDa; the degree of caproyl substitution is 50%; the caproyl modified chitosan is dissolved in normal saline; and the concentration is 5g/L) in a volume ratio of 1:10 (i.e., the microspheres : each solution (v/v)) in sequence, allow them to react for 20 min, wash with normal saline, then allow the washed product to react with 2g/L of sodium alginate solution for 10 min, and wash with the saline to obtain the ACC_{caproyl}A microcapsules.
3) After mixing the ACC_{caproyl}A microcapsules encapsulating CHO cells containing recombinant endostatin with 50% (V/V) of glycerol solution with an apparent viscosity of 600 cp (25°C) in a volume ratio of 1:5, transplant the mixture into the abdominal cavity of the melanoma model rat. The results show that, the melanoma of the rat obviously becomes small; the ACC_{caproyl}A microcapsules keep intact, and the surface is free from fibrous encapsulation, when they are recovered two months after transplantation.

### Example 8

1) Prepare the calcium alginate gel microspheres encapsulating the porcine islet cells by a high-voltage electrostatic method, wherein each microsphere contains 1-2 islet cells.
2) Soak the microspheres into the chitosan solution (the molecular weight of the chitosan is 40kDa; the degree of deacetylation is 98%; the chitosan is dissolved in the acetic acid-sodium acetate buffer solution with a pH of 6.5; and the concentration is 5g/L), 0.2% of sodium alginate solution and acetyl modified chitosan solution (the molecular weight of the chitosan framework is 60kDa; the degree of acetyl substitution is 50%; the acetyl modified chitosan is dissolved in normal saline; and the concentration is 5g/L) in a volume ratio of 1:10 (i.e., the microspheres : each solution (v/v)) in sequence, allow them to react for 20 min, wash with normal saline, then allow the washed product to react with 2g/L of sodium alginate solution for 10 min, wash with normal saline, liquefy with 55mM of sodium citrate solution, and wash with normal saline to obtain the ACC_{acetyl}A microcapsules.
2) After mixing the resultant ACC_{acetyl}A microcapsules with the polyethylene glycol solution (100g/L) with an apparent viscosity of 400 cp (25°C) in a volume ratio of 1:2, apply the mixture for cell therapy of the diabetes model rat. By transplantation into the abdominal cavity, the blood sugar level of the rat returns to normal only one day after the transplantation, and the diabetes symptom is significantly alleviated; when they are recovered six months after *in vivo* transplantation, it is found that, the microcapsules are intact; the microcapsule surface is smooth and free from fibrous encapsulation; and the islet cells in the microcapsules keep alive with positive dithizone staining.

## Claims

1. A microcapsule preparation of alginate-chitosan acyl derivatives, comprising microcapsules of alginate-chitosan acyl derivatives or formed by mixing microcapsules of alginate-chitosan acyl derivatives with aqueous solution, wherein: the biomicrocapsule structure consists of two parts, a microcapsule membrane and an inner core; the microcapsule membrane is a polyelectrolyte composite hydrogel membrane formed by chitosan, alginates and chitosan acyl derivatives, and the inner core is an alginate liquid or a hydrogel environment containing cells.

2. The biomicrocapsule preparation according to claim 1, **characterized in that**, in the preparation, the microcapsules are spherical microcapsules with a particle size of 10 to 2,000 µm; the membrane thickness is 0.1 to 100 µm, and the molecular weight of the alginate forming the membrane is 10 kDa to 2,000 kDa; the chitosan material has a degree of deacetylation of 70 to 98%, and molecular weight of 1 kDa to 500 kDa; the molecular weight of the chitosan acyl derivatives is 1kDa to 800kda; the mass ratio of the chitosan, alginate and chitosan acyl derivatives is 0:1:0.1 to 10:1:10; and the alginate concentration in the core is 0.1 to 50g/L.

3. The biomicrocapsule preparation according to claim 1 or 2, **characterized in that**, in the preparation, the chitosan acyl derivatives in the microcapsule are N-acyl chitosan, with a monomer structure as below: 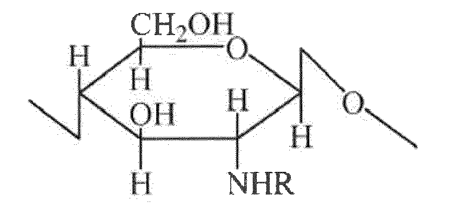 wherein, -R represents formyl, acetyl, propionyl, butyryl, valeryl or caproyl; the substitution value of the acyl derivatives is 10 to 60%; the molecular weight of the chitosan framework material is 1 to 400 kDa; and the degree of deacetylation is 90 to 98%.

4. The biomicrocapsule preparation according to claim 1 or 2, **characterized in that**: in the preparation, the alginate as a component of the microcapsule membrane is potassium or sodium alginate.

5. The biomicrocapsule preparation according to claim 1 or 2, **characterized in that**: in the preparation, the alginate gel in the inner core of the microcapsule is alginate hydrogel of one or two or more of divalent calcium, barium and zinc, and the alginate liquid is the solution of potassium or sodium alginate.

6. The biomicrocapsule preparation according to claim 1, **characterized in that**: in the biomicrocapsule preparation, the volume ratio of the biomicrocapsule to the aqueous solution is 10:1 to 1:100, wherein the aqueous solution is one or a mixture of two or more of normal saline, HEPES solution, hyaluronic acid solution with an apparent viscosity of 5 to 2,000cp (25°C), the sodium alginate with an apparent viscosity of 5 to 2,000cp (25°C), the glucosan solution with an apparent viscosity of 5 to 2,000cp (25°C), glycerol solution with an apparent viscosity of 5 to 2,000cp (25°C), polyethylene glycol solution with an apparent viscosity of 5 to 2,000cp (25°C), polyvinylpyrrolidone solution with an apparent viscosity of 5 to 2,000cp (25°C), cellulose derivative solution with an apparent viscosity of 5 to 2,000cp (25°C), cyclodextrin solution with an apparent viscosity of 5 to 2,000cp (25°C), starch solution with an apparent viscosity of 5 to 2,000cp (25°C), and starch derivative solution with an apparent viscosity of 5 to 2,000cp (25°C).

7. A method for preparing the biomicrocapsule preparation according to claim 1, **characterized in that**, the microcapsule membrane is a hydrogel membrane formed by chitosan, alginate, chitosan acyl derivatives through polyelectrolyte complexation reaction; the preparation steps of the biomicrocapsule preparation are as follows: under the sterilized conditions,
1) preparing alginate gel microspheres encapsulating living cells, called microspheres A;
2) soaking the microspheres A obtained in step 1) into the chitosan solution in a volume ratio 1:1 to 1:40 (i.e., microspheres A : chitosan solution (v/v)), allowing them to react for 1 to 60 min to obtain sodium alginate-chitosan microcapsules called microspheres B, and separating and washing the microspheres B with normal saline; wherein
the chitosan solution is prepared by dissolving the chitosan in the acetic acid-sodium acetate buffer solution with a pH of 5.5 to 7.0, and the chitosan concentration is 0.1 to 15 g/L;
3) soaking the microspheres B obtained in step 2) into alkaline metal alginate solution (the alginate concentration is 0.1 to 5 g/L) in a volume ratio of 1:1 to 1:40 (i.e., microspheres B : alkaline metal alginate solution (v/v)), allowing them to react for 1 to 60min to obtain microcapsules called microspheres C, and separating and washing the microspheres C with saline;
4) repeating step 2) and step 3) for 1-5 cycles to obtain microcapsules called microspheres D, and separating and washing the microspheres D with normal saline;
5) soaking the microspheres A, B, C or D respectively obtained in step 1), 2), 3) or 4) into the chitosan acyl derivative solution in a volume ratio of 1:1 to 1:40 (i.e., microspheres : chitosan acyl derivative solution (v/v)), allowing them to react for 1 to 60 min to obtain microcapsules having an inner gel core, called microspheres E, and separating and washing the microspheres E with the saline, wherein
the chitosan acyl derivative solution is prepared by dissolving the chitosan acyl derivatives in normal saline, HEPES buffer solution, PBS buffer solution or acetic acid-sodium acetate buffer solution with a pH of 5.5 to 7.0, and the chitosan acyl derivative concentration is 0.1 to 20 g/L;
6) soaking the microspheres E obtained in step 5) into the alkaline metal alginate solution, and repeating step 3) to obtain microcapsules with the neutral surface and the inner gel core, called microspheres F;
7) soaking the microspheres F obtained in step 6) into the organic metal chelating agent solution in a volume ratio of 1:1 to 1:40 (i.e., microspheres F : the organic metal chelating agent solution (v/v)) to liquefy the alginate gel in the microcapsules, allowing them to react for 1 to 60 min, seperating the product, washing it with normal saline to obtain the microcapsules having an inner liquid core, called microspheres G;
8) mixing the microspheres E, F or G respectively obtained in step 5), 6) or 7) with aqueous solution to obtain the microcapsule preparation of alginate-chitosan acyl derivatives.

8. The method for preparing the microcapsules according to claim 7, **characterized in that**, the alginate gel microspheres are alginate hydrogel of one or two or more of divalent calcium, barium or zinc;
and the alkaline metal alginate for neutralizing the surface charges in step 3) and step 6) is potassium or sodium alginate with a molecular weight of 10kDa to 2,000kDa and a concentration of 0.1 to 5g/L.

9. The method for preparing the microcapsules according to claim 7, **characterized in that**, the organic chelating agent solution involved in the liquefying reaction is 40 to 70mmol/L of sodium citrate or 50 to 200mmol/L of EDTA.

10. Use of the claim 1, **characterized in that**, the microcapsules in the preparation are used for cell encapsulation.

11. The use according to claim 10, **characterized in that**, the cells are *ex vivo* or *in vitro* cells coming from human or mammals, such as islet cells, liver cells, thyroid cells, parathyroid cells, adrenal chromaffin cells, cells capable of secreting bioactive substances, cell lines cells, genetically engineered cells, stem cells or various differentiated cells from stem cells.
